Europäisches Patentamt

European Patent Office

Office européen des brevets

(11) Veröffentlichungsnummer : **0 037 511**
**B1**

(12) **EUROPÄISCHE PATENTSCHRIFT**

(45) Veröffentlichungstag der Patentschrift :
16.02.83

(51) Int. Cl.³ : **C 07 C143/60**

(21) Anmeldenummer : **81102196.3**

(22) Anmeldetag : **24.03.81**

(54) **Verfahren zur Isolierung von 1-Naphthylamin-4.6- und 1-Naphthylamin-4.7-disulfonsäure.**

(30) Priorität : **05.04.80 DE 3013276**

(43) Veröffentlichungstag der Anmeldung :
**14.10.81 Patentblatt 81/41**

(45) Bekanntmachung des Hinweises auf die Patenterteilung : **16.02.83 Patentblatt 83/07**

(84) Benannte Vertragsstaaten :
**CH DE FR GB IT LI**

(56) Entgegenhaltungen :
**CHEMICAL ABSTRACTS, Band 63, Nr. 7, 27. September 1965, Spalte 8288b-d Columbus, Ohio, U.S.A.**
**CHEMICAL ABSTRACTS, Band 71, Nr. 9, 1. September 1969, Seite 258, Nr. 38659a Columbus, Ohio, U.S.A.**

(73) Patentinhaber : **BAYER AG**
**Zentralbereich Patente, Marken und Lizenzen**
**D-5090 Leverkusen 1, Bayerwerk (DE)**

(72) Erfinder : **Blank, Heinz Ulrich, Dr.**
**Am Geusfelde 35**
**D-5068 Odenthal (DE)**
Erfinder : **Behre, Horst, Dr.**
**Zur alten Linde 12**
**D-5068 Odenthal-Eikamp (DE)**
Erfinder : **Linden, Hans Werner, Dr.**
**Heymannstrasse 38**
**D-5090 Leverkusen (DE)**

Verfahren zur Isolierung von 1-Naphthylamin-4.6- und 1-Naphthylamin-4.7-disulfonsäure

Die Erfindung betrifft ein Verfahren zur Isolierung von 1-Naphthylamin-4.6- und 1-Naphthylamin-4.7-disulfonsäure aus dem bei der Sulfonierung von 1-Naphthylamin-6- bzw. 1-Naphthylamin-7-sulfonsäure anfallenden Reaktionsgemisch.

1-Naphthylamin-4.6- und 1-Naphthylamin-4.7-disulfonsäure sind wichtige Zwischenprodukte für die Herstellung von Farbstoffen. Sie werden technisch durch Sulfonieren von 1-Naphthylamin-6- bzw. 1-Naphthylamin-7-sulfonsäure hergestellt (s. Ullmanns Enzyklopädie der technischen Chemie 4. Auflage, Band 17, Seite 112 (1979)). Zum Entfernen unerwünschter Nebenprodukte ist eine Isolierung der beiden Disulfonsäuren aus den Sulfonierungsgemischen erforderlich. Üblicherweise werden 1-Naphthylamin-4.6- und 1-Naphthylamin-4.7-disulfonsäure als saure Alkalisalze isoliert. Dies geschieht durch Eintragen der jeweiligen Sulfonierungsgemische in Wasser, Aussalzen mittels Kochsalz (s. N. Donaldson « The Chemistry and Technology of Naphthalene Compounds » 1958, Seite 203) oder Natriumsulfat (s. CSSR-Patentschrift Nr. 129 210), Abfiltrieren und Waschen der auskristallisierten Salze. 1-Naphthylamin-4.6-disulfonsäure kann auch als schwerlösliches Magnesiumsalz abgetrennt werden (s. CSSR-Patentschrift Nr. 112 946).

Diese Isolierung der sauren Alkalisalze der 1-Naphthylamin-4.6- bzw. -4.7-disulfonsäuren ist jedoch mit erheblichen Schwierigkeiten verbunden, weil die Salze in Form eines feinen Kristallbreies, häufig sogar als thixotrope Massen, anfallen, und sich nur schlecht abfiltrieren und waschen lassen. Außerdem enthalten die abfiltrierten Sulfonsäuresalze störende Mengen an Salz, z. B. Kochsalz.

Ein weiterer Nachteil dieser bekannten Arbeitsweisen ist ferner, daß große Mengen Salzlösung, z. B. Kochsalzlösung, zum Nachwaschen benötigt werden und daß die nach dem Abfiltrieren der sulfonsauren Salze anfallende Dünnsäure erhebliche Salzmengen enthält, die eine Wiederaufbereitung der Dünnsäure erschweren.

Es wurde nun gefunden, daß sich die vorstehend aufgezeigten Nachteile bei der Isolierung der 1-Naphthylamin-4.6- bzw. -4.7-disulfonsäure vermeiden lassen, wenn man die beim Eintragen der jeweiligen Sulfonierungsgemische in Wasser entstehenden wäßrigen Lösungen bzw. Suspensionen auf 80 bis 120 °C vorzugsweise 90 bis 115 °C erwärmt bzw. sich erwärmen läßt, auf das Aussalzen verzichtet und statt dessen aus den 80 bis 120 °C warmen wäßrigen Lösungen bzw. Suspensionen der Sulfonierungsgemische die freien Naphthylamindisulfonsäuren durch Abkühlen ausscheidet.

Bei dieser Arbeitsweise kristallisieren die freien 1-Naphthylamin-4.6- bzw. 1-Naphthylamin-4.7-disulfonsäuren in hoher Ausbeute und Reinheit in Form gut filtrierbarer Kristallniederschläge aus. Zum Auswaschen der abfiltrierten Kristallniederschläge wird nur wenig verdünnte Schwefelsäure benötigt. Die nach dem Abfiltrieren der 1-Naphthylamin-4.6- bzw. 1-Naphthylamin-4.7-disulfonsäure anfallende Dünnsäure ist salzfrei und daher ohne Schwierigkeiten aufzuarbeiten.

Die Erfindung betrifft daher ein Verfahren zur Isolierung von 1-Naphthylamin-4.6- bzw. 1-Naphthylamin-4.7-disulfonsäure aus bei der Sulfonierung von 1-Naphthylamin-6- bzw. 1-Naphthylamin-7-sulfonsäure mit Schwefelsäure und Schwefeltrioxid angefallenen Sulfonierungsgemischen ; das Verfahren ist dadurch gekennzeichnet, daß man die beim Eintragen der Sulfonierungsgemische in Wasser entstehenden wäßrigen Lösungen bzw. Suspensionen auf 80 bis 120 °C vorzugsweise 90 bis 115 °C erwärmt und die entstandenen Lösungen bzw. Suspensionen der Sulfonierungsgemische, gegebenenfalls nachdem man sie eine Zeit lang auf diesen Temperaturen gehalten hat, auf Temperaturen unter 70 °C, vorzugsweise von 20 bis 60 °C abkühlt und die auskristallisierte 1-Naphthylamin-4.6- bzw. 1-Naphthylamin-4.7-disulfonsäure abfiltriert.

Zum erfindungsgemäßen Erwärmen der wäßrigen Lösungen bzw. Suspensionen der Sulfonierungsgemische auf 90 bis 120 °C nutzt man vorteilhaft die beim Eintragen der Sulfonierungsgemische in Wasser freiwerdende Verdünnungswärme. Das heißt man trägt vorteilhaft die Sulfonierungsgemische so rasch in das vorgelegte Wasser ein, daß die Temperatur der entstehenden Lösungen bzw. Suspensionen ohne zusätzliches Erwärmen bzw. ohne Kühlen auf 80 bis 120 °C ansteigt. Trägt man das Sulfonierungsgemisch langsamer ein, muß gegebenenfalls erwärmt werden, wählt man die Eintragsgeschwindigkeit höher, muß gegebenenfalls gekühlt werden.

Für die Isolierung der 1-Naphthylamin-4.6-disulfonsäure hat sich ein Erwärmen der wäßrigen Lösungen bzw. Suspensionen der Sulfonierungsgemische auf 90 bis 110 °C, für die Isolierung der 1-Naphthylamin-4.7-disulfonsäure ein Erwärmen auf Temperaturen von 100 bis 115 °C, besonders bewährt.

Die vorzulegende Wassermenge, in die das Sulfonierungsgemisch eingetragen wird, wird vorteilhaft so bemessen, daß die Schwefelsäurekonzentration in der verdünnten wäßrigen Lösung bzw. Suspension der Sulfonierungsgemische etwa 10 bis 70 Gew.-%, vorzugsweise 25 bis 60 Gew.-% beträgt. Für die Isolierung der 1-Naphthylamin-4.6-disulfonsäure hat sich insbesondere eine Schwefelsäurekonzentration von 40 bis 60 Gewichts-%, für die Isolierung der 1-Naphthylamin-4.7-disulfonsäure eine Schwefelsäurekonzentration von 25 bis 40 Gewichts-%, bewährt.

Die auskristallisierten 1-Naphthylamin-disulfonsäuren werden vorteilhaft bei Temperaturen von 20 bis 60 °C abfiltriert.

Das erfindungsgemäße Verfahren eignet sich zur Isolierung von 1-Naphthylamin-4.6- und 1-Naphthylamin-4.7-disulfonsäure aus allen bei der Sulfonierung von 1-Naphthylamin-6- bzw. 1-Naphthylamin-7-

0 037 511

sulfonsäuren mit Schwefelsäure und Schwefeltrioxid anfallenden Sulfonierungsgemischen, unabhängig von den bei der Sulfonierung angewandten Reaktionsbedingungen.

Das erfindungsgemäße Verfahren kann beispielsweise wie folgt durchgeführt werden : In einer Rührapparatur wird das zum Verdünnen des Sulfonierungsgemisches erforderliche Wasser vorgelegt. Dann wird das Sulfonierungsgemisch unter Rühren so rasch eingetragen, daß sich die entstehende Lösung bzw. Suspension auf eine Temperatur von 80 bis 120 °C erwärmt und, falls eine Suspension entsteht, diese jederzeit gut rührbar bleibt. Wird die innerhalb des Bereichs von 90 bis 120 °C gewünschte Temperatur nicht schon bereits durch die Verdünnungswärme erreicht, so wird die erhaltene wäßrige Lösung bzw. Suspension nach beendetem Eintragen auf die gewünschte Temperatur innerhalb des angegebenen Bereiches erwärmt. Besonders im Falle der 1-Naphthylamin-4.7-disulfonsäure hat es sich bewährt, die entstandene wäßrige Lösung bzw. Suspension je eine Stunde bei 110 °C, 100 °C und 90 °C nachzurühren. Anschließend wird die heiße Lösung bzw. Suspension langsam unter Rühren auf Temperaturen unter 70 °C, vorzugsweise 20 bis 60 °C abgekühlt. Der ausgefallene Kristallniederschlag wird abgesaugt und mit wenig verdünnter Schwefelsäure (z. B. 40 bis 60 %iger Schwefelsäure) gewaschen.

Die auf diese Weise erhaltenen 1-Naphthylamin-disulfonsäuren zeichnen sich durch hohe Reinheit aus ; ihr Gehalt an Nebenprodukten liegt unter 2 Gew.-%.

## Beispiel 1

In einer Rührapparatur werden 1 650 g Wasser vorgelegt und 1 171 g des nachstehend beschriebenen Sulfonierungsgemisches so rasch zugegeben, daß die sich bildende Suspension gut rührbar bleibt. Die Temperatur steigt während des Eintragens auf 95 bis 100 °C. Anschließend wird die wäßrige Suspension unter Rühren auf etwa 110 °C erwärmt, eine Stunde auf dieser Temperatur gehalten, dann je eine Stunde bei 100 °C und 90 °C gerührt. Anschließend wird langsam unter Rühren auf 20 °C abgekühlt. Der Niederschlag wird abfiltriert und 2-mal mit wenig 35 %iger Schwefelsäure gewaschen.

Es werden 460 g 1-Naphthylamin-4.7-disulfonsäure in Form schwefelsäurefeuchter schwach grauer Kristalle erhalten.

Die Zusammensetzung des Produktes wurde durch Hochdruckflüssigkeitschromatographie und Dünnschichtchromatographie bestimmt. Sie beträgt : 61,5 Gew.-% 1-Naphthylamin-4.7-disulfonsäure ; 0,05 Gew.-% 1-Naphthylamin-7-sulfonsäure ; 0,05 Gew.-% 1-Naphthylamin-2.4.7-trisulfonsäure ; etwa 0,7 Gew.-% unbekannte Verunreinigungen ; Rest bis 100 % Schwefelsäure und Wasser.

Das verwendete Sulfonierungsgemisch war wie folgt erhalten worden :

In einer 1 l Rührapparatur wurden 700 g Monohydrat vorgelegt und 225 g 1-Naphthylamin-7-sulfonsäure (etwa 99 %ig) eingetragen. Anschließend wurden bei 30 °C 246 g Oleum (65 %ig) innerhalb von 30 Minuten zugetropft und die Sulfonierungsmischung etwa 8 Stunden bei 30 °C gerührt.

## Beispiel 2

Es wird wie in Beispiel 1 beschrieben verfahren, nur werden statt der 1 650 g Wasser 1 550 g vorgelegt und ein Sulfonierungsgemisch verwendet, das wie nachstehend beschrieben erhalten wurde.

Es werden 505 g 1-Naphthylamin-4.7-disulfonsäure in Form schwefelsäurefeuchter, schwach grauer Kristalle erhalten.

Die Zusammensetzung des Produktes wurde durch Hochdruckflüssigkeitschromatographie und Dünnschichtchromatographie bestimmt. Sie beträgt :

54,6  Gew.-% 1-Naphthylamin-4.7-disulfonsäure ;
0,05 Gew.-% 1-Naphthylamin-7-sulfonsäure ;
0,05 Gew.-% 1-Naphthylamin-2.4.7-trisulfonsäure ;
etwa
1,5  Gew.-% unbekannte Verunreinigungen ;
        Rest bis 100 % Schwefelsäure und Wasser.

Das verwendete Sulfonierungsprodukt war wie folgt erhalten worden :
In einer 1 l Rührapparatur wurden 700 g Monohydrat vorgelegt und 225 g 1-Naphthylamin-7-sulfonsäure (etwa 99 %ig) eingetragen. Dann wurden bei 30 °C 185 g Oleum (65 %ig) zugegeben und die Sulfonierungsmischung etwa 24 Stunden bei 30 °C gerührt.

## Beispiel 3

In einer Rührapparatur werden 1 920 g Wasser vorgelegt und 1 518 g des nachstehend beschriebenen Sulfonierungsgemisches so rasch unter Rühren eingetragen, daß sich die entstehende Suspension auf 100 bis 105 °C erwärmt. Anschließend wird die Suspension auf 110 °C erwärmt und je eine Stunde bei 110 °C, 100 °C und 90 °C gehalten. Dann wird die Suspension langsam auf 60 °C abgekühlt, eine Stunde auf dieser Temperatur gehalten, anschließend bei 60 °C filtriert. Der Filterkuchen wird mit wenig 40 %iger

3

Schwefelsäure gewaschen. Es werden 458 g 1-Naphthylamin-4.7-disulfonsäure in Form schwefelsäurefeuchter, schwach grauer Kristalle erhalten.

Die Zusammensetzung des Produktes wurde durch Hochdruckflüssigkeitschromatographie und Dünnschichtchromatographie bestimmt. Sie beträgt :

50,6 Gew.-% 1-Naphthylamin-4.7-disulfonsäure ;
0,05 Gew.-% 1-Naphthylamin-7-sulfonsäure ;
0,05 Gew.-% 1-Naphthylamin-2.4.7-trisulfonsäure ;
etwa
1,5 Gew.-% unbekannte Verunreinigungen ;
Rest bis 100 % Wasser und Schwefelsäure.

Das verwendete Sulfonierungsgemisch war wie folgt erhalten worden :
In einer 1 l Rührapparatur wurden 713 g Monohydrat vorgelegt und 225 g 1-Naphthylamin-7-sulfonsäure (etwa 99 %ig) eingetragen. Dann wurden bei 30-35 °C 580 g Oleum (65 %ig) zugegeben und die Sulfonierungsmischung etwa 14 Stunden bei 25 °C gerührt.

Beispiel 4

In einer Rührapparatur werden 1 434 g Wasser vorgelegt und 1 232,8 g des nachstehend beschriebenen Sulfonierungsgemisches innerhalb von 10 Minuten eingetragen. Die Temperatur steigt dabei auf etwa 95 °C ; nach kurzer Zeit beginnt die 1-Naphthylamin-4.6-disulfonsäure auszukristallisieren. Dann läßt man unter schwachem Rühren innerhalb von etwa 6 Stunden auf etwa 20 °C abkühlen, filtriert vom Kristallniederschlag ab und wäscht diesen zweimal mit je 100 ml 40 %iger Schwefelsäure. Der Filterkuchen wird gut trocken gesaugt.

Auf diese Weise werden 321 g 1-Naphthylamin-4.6-disulfonsäure in Form schwefelsäurefeuchter, schwach rosa gefärbter Kristalle erhalten.

Die Zusammensetzung des Produktes wurde durch Hochdruckflüssigkeitschromatographie und Dünnschichtchromatographie bestimmt. Sie beträgt :

76,1 Gew.-% 1-Naphthylamin-4.6-disulfonsäure ;
0,1 Gew.-% 1-Naphthylamin-3,6-disulfonsäure ;
Spur     1-Naphthylamin-2.4.6-trisulfonsäure ;
23,7 Gew.-% Schwefelsäure/Wasser.

Das verwendete Sulfonierungsgemisch war wie folgt erhalten worden :
In einer 1 l Rührapparatur wurden 226,8 g 1-Naphthylamin-6-sulfonsäure (98,3 %ig) in 760 g vorgelegtes Monohydrat eingetragen. Dann wurden bei 30 °C innerhalb von 30 Minuten 246 g Oleum (65 %ig) zugetropft. Anschließend wurde die Sulfonierungsmischung 16 Stunden bei 30 °C nachgerührt.

Beispiele 5 bis 7

Es wurde wie in Beispiel 1 beschrieben gearbeitet, nur wurden statt der 1 434 g Wasser in

Beispiel 5     962 g Wasser,
Beispiel 6     647 g Wasser,
Beispiel 7     647 g Wasser.

vorgelegt und auf diese Weise verschiedene Schwefelsäurekonzentrationen in den wäßrigen Lösungen bzw. Suspensionen der Sulfonierungsgemische eingestellt. Außerdem wurde die auskristallisierte 1-Naphthylamin-4,6-disulfonsäure in

Beispiel 5 bei 40 °C,
Beispiel 6 bei 40 °C,
Beispiel 7 bei 60 °C.

abgesaugt und zweimal mit je 100 ml 50 %iger Schwefelsäure gewaschen.
In der nachstehenden Tabelle ist die Zusammensetzung der unter den verschiedenen Bedingungen isolierten 1-Naphthylamin-4,6-disulfonsäure angegeben.

(Siehe die Tabelle, Seite 5)

## Tabelle

| Beispiel Nr. | Menge an vorgelegtem Wasser | Gew.-% H$_2$SO$_4$ in der wäßr. Lösg. bzw. Suspension | Absaug-temp. °C | Ausbeute an krist. Produkt g | Zusammenzetzung des krist. Produkts (in Gew.-%)* | | | |
|---|---|---|---|---|---|---|---|---|
| | | | | | 1-Naphthylamin-4.6-disulfonsäure | 1-Naphthylamin-3.6-disulfonsäure | 1-Naphthylamin-2.4.6 trisulfonsäure | H$_2$O/H$_2$SO |
| 5 | 962 | 50 | 40 | 311,7 | 80,0 | 0,2 | 0,1 | 19,7 |
| 6 | 647 | 60 | 40 | 327 | 73,3 | 0,4 | 0,2 | 26,1 |
| 7 | 647 | 60 | 60 | 310,4 | 77,5 | 0,4 | Spur | 22,0 |

* Die Zusammensetzung der kristallisierten Produkte wurde mittels Hochdruckflüssigkeitschromatographie und Dünnschichtchromatographie bestimmt.

## Beispiel 8

In einer Rührapparatur werden 1 090 g Wasser vorgelegt und 1 005 g des nachstehend beschriebenen Sulfonierungsgemisches innerhalb von 10 Minuten eingetragen. Die Temperatur steigt dabei auf 95 °C. Nach kurzer Zeit beginnt die 1-Naphthylamin-4.6-disulfonsäure auszukristallisieren. Man läßt unter schwachem Rühren langsam auf 60 °C abkühlen, hält eine Stunde bei dieser Temperatur und filtriert die 60 °C heiße Suspension. Der Filterkuchen wird mit wenig 40 %iger Schwefelsäure gewaschen und gut trockengesaugt.

Es werden 217 g 1-Naphthylamin-4.6-disulfonsäure in Form schwefelsäurefeuchter, schwach rosa gefärbter Kristalle erhalten.

Die Zusammensetzung der Kristalle wurde durch Hochdruckflüssigkeitschromatographie und Dünnschichtchromatographie bestimmt. Die Zusammensetzung beträgt :

65,0 Gew.-% 1-Naphthylamin-4,6-disulfonsäure ;
0,5 Gew.-% 1-Naphthylamin-3,6-disulfonsäure ;
0,5 Gew.-% 1-Naphthylamin-2.4.6-trisulfonsäure ;
34,0 Gew.-% Wasser/Schwefelsäure.

Das verwendete Sulfonierungsgemisch war wie folgt erhalten worden :
In einer 1 l Rührapparatur wurden 780 g Oleum (25 %ig) vorgelegt. In dieses wurden bei 20 °C in ca. 30 Minuten 225 g 1-Naphthylamin-6-sulfonsäure (ca. 99 %ig) eingetragen. Anschließend wurde die Sulfonierungsmischung 4 Stunden bei 50 °C gerührt.

## Ansprüche

1. Verfahren zur Isolierung von 1-Naphthylamin-4.6-bzw. 1-Naphthylamin-4.7-disulfonsäure aus bei der Sulfonierung von 1-Naphthylamin-6- bzw. 1-Naphthylamin-7-sulfonsäure mit Schwefelsäure und Schwefeltrioxid angefallenen Sulfonierungsgemischen, dadurch gekennzeichnet, daß man die beim Eintragen der Sulfonierungsgemische in Wasser entstehenden wäßrigen Lösungen bzw. Suspensionen auf 80 bis 120 °C erwärmt und die entstandenen Lösungen bzw. Suspensionen, gegebenenfalls nachdem man sie eine Zeit auf dieser Temperatur gehalten hat, auf Temperaturen unter 70 °C abkühlt und die auskristallisierte 1-Naphthylamin-4.6- bzw. 1-Naphthylamin-4.7-disulfonsäure abfiltriert.

2. Verfahren gemäß Anspruch 1, dadurch gekennzeichnet, daß man die beim Eintragen der Sulfonierungsgemische in Wasser entstehenden wäßrigen Lösungen bzw. Suspensionen auf 90 bis 115 °C erwärmt.

3. Verfahren gemäß Anspruch 1 und 2, dadurch gekennzeichnet, daß die Schwefelsäurekonzentration in den verdünnten wäßrigen Lösungen bzw. Suspensionen der Sulfonierungsgemische 10 bis 70 Gew.-% beträgt.

4. Verfahren gemäß Anspruch 1 bis 3, dadurch gekennzeichnet, daß die Schwefelsäurekonzentration in den verdünnten wäßrigen Lösungen bzw. Suspensionen der Sulfonierungsgemische 25 bis 60 Gew.-% beträgt.

## Claims

1. Process for the isolation of 1-naphthylamine-4,6- and 1-naphthylamine-4,7-disulphonic acid from sulphonation mixtures obtained in the sulphonation of 1-naphthylamine-6- or 1-naphthylamine-7-sulfonic acid with sulphuric acid and sulphur trioxide, characterised in that the aqueous solutions or suspensions formed when the sulphonation mixtures are introduced into water are warmed to 80 to 120 °C and the resulting solutions or suspensions are cooled to temperatures below 70 °C, if necessary after they have been kept at the higher temperature for a period, and the 1-naphthylamine-4,6- or 1-naphthylamine-4,7-disulphonic acid which has crystallised out is filtered off.

2. Process according to Claim 1, characterised in that the aqueous solutions or suspensions formed when the sulphonation mixtures are introduced into water are warmed to 90 to 115 °C.

3. Process according to Claims 1 and 2, characterised in that the sulphuric acid concentration in the dilute aqueous solutions or suspensions of the sulphonation mixtures is 10 to 70 % by weight.

4. Process according to Claims 1 to 3, characterised in that the sulphuric acid concentration in the dilute aqueous solutions or suspensions of the sulphonation mixtures is 25 to 60 % by weight.

**Revendications**

1. Procédé pour isoler l'acide 1-naphtylamine-4.6- ou 1-naphtylamine-4.7-disulfonique de mélanges sulfonés obtenus dans la sulfonation de l'acide 1-naphtylamine-6- et 1-naphtylamine-7-sulfonique par l'acide sulfurique et l'anhydride sulfurique, caractérisé en ce qu'on chauffe à 80-120 °C les solutions ou suspensions aqueuses obtenues lors de l'introduction des mélanges à sulfoner dans de l'eau et on refroidit à des températures inférieures à 70 °C les solutions ou suspensions formées, éventuellement après les avoir maintenues pendant un certain temps à la température de chauffage, et on filtre l'acide 1-naphtylamine-4.6- ou 1-naphtylamine-4.7-disulfonique cristallisé.

2. Procédé suivant la revendication 1, caractérisé en ce qu'on chauffe à 90-115 °C les solutions ou suspensions aqueuses obtenues en introduisant les mélanges à sulfoner dans l'eau.

3. Procédé suivant les revendications 1 et 2, caractérisé en ce que la concentration en acide sulfurique dans les solutions ou suspensions aqueuses diluées des mélanges à sulfoner est de 10 à 70 % en poids.

4. Procédé suivant les revendications 1 à 3, caractérisé en ce que la concentration en acide sulfurique dans les solutions ou suspensions aqueuses diluées des mélanges à sulfoner est de 25 à 60 % en poids.